# EUROPEAN PATENT APPLICATION

(11) **EP 4 778 441 A1**
(43) Date of publication of application: **22.07.2026**
(21) Application number: 24864968.3
(22) Date of filing: 14.03.2024
(51) Int. Cl.: A61B 1/00, A61B 1/05

(54) **ENDOSCOPE**

(30) Priority: 13.09.2023 US 202363538160 P
(71) Applicant: Olympus Medical Systems Corp., Tokyo 192-8507 (JP)
(72) Inventor: YOSHINAGA, Takuto, Hachioji-shi, Tokyo 192-8507 (JP)
(74) Representative: Schicker, Silvia
(86) International application number: PCT/JP2024/010129
(87) International publication number: WO 2025/057458

(57) **Abstract**

An endoscope includes a distal end barrel, a fitted portion that is provided on a proximal end side of the distal end barrel and has an outer diameter dimension smaller than an outer diameter dimension on a distal end side of the distal end barrel, a coupling ring fitted into the fitted portion, a recess portion provided in the coupling ring, a convex portion that protrudes from a part of the fitted portion in a radial direction of the distal end barrel and engages with the recess portion, and an image pickup module that is incorporated in the distal end barrel in a vicinity of the convex portion and is disposed in a region overlapping the convex portion in the radial direction of the distal end barrel.

## Description

### Technical Field

The present disclosure relates to an endoscope in which a coupling member is fitted into a proximal end side of a distal end portion.

### Background Art

Conventionally, endoscopes are widely used in medical fields, industrial fields, and the like. The endoscope can perform observation, treatment, and the like in a subject by inserting an insertion portion into the subject.

A distal end portion of the insertion portion is configured by a rigid distal end barrel. The distal end barrel includes a plurality of through holes that penetrate in a longitudinal axis direction of the insertion portion. Members such as an image pickup module, an illumination optical system, a forceps channel tube, and the like are inserted into the through holes, respectively. The members are fixed in a liquid-tight manner to the through holes respectively by an adhesive or the like.

A proximal end side of such a distal end barrel is coupled with a bending portion via a coupling ring. Here, the coupling ring is adhesively fixed to the distal end barrel in a fitted state. In order to achieve the fitting of the distal end barrel and the coupling ring, for example, International Publication No. 2021/175093 discloses a technology in which a bonding portion (fitted portion) having a circumferential surface with an outer diameter dimension smaller than that of an outer circumferential surface on a distal end side of the distal end barrel is formed at a proximal end portion of the distal end barrel.

However, when the proximal end portion of the distal end barrel is formed with the circumferential surface with the outer diameter dimension smaller than that of the distal end barrel on the distal end side in order to achieve the fitting of the distal end barrel and the coupling ring, a layout for installing each member such as the image pickup module in the distal end barrel is restricted. In other words, when the proximal end portion of the distal end barrel is formed with the circumferential surface with the outer diameter smaller than that of the distal end barrel on the distal end side, it is necessary to lay out each through hole for inserting each member on an inner side with respect to the circumferential surface of the proximal end portion of the distal end barrel.

On the other hand, by cutting out a part of a wall of each through hole on the proximal end side, it is possible to relax the restrictions of the layout of each member with respect to the distal end barrel without increasing the overall outer diameter dimension of the distal end barrel.

At the same time, when cutting out the part of the wall of each through hole, the skill required for a worker is high when the image pickup module is adhesively fixed to the through hole, in particular. In other words, when the part of the through hole is cut out, the worker is required to have a high level of skill in adhesively fixing the image pickup module to the through hole in the liquid-tight manner. In addition, when the part of the through hole is cut out, the worker is required to have a high level of skill in positioning an optical axis direction of the image pickup module along a wall surface of the through hole.

The present disclosure has been made in view of the above circumstances, and its object is to provide an endoscope capable of adhesively fixing an image pickup module to a distal end barrel accurately, without increasing an overall outer diameter dimension of the distal end barrel.

### Disclosure of Invention

### Means for Solving the Problem

An endoscope according to an aspect of the present disclosure includes: a distal end barrel constituting a distal end portion of an insertion portion; a fitted portion that is provided on a proximal end side of the distal end barrel and has an outer diameter dimension smaller than an outer diameter dimension on a distal end side of the distal end barrel; a coupling member fitted into the fitted portion; a recess portion provided in the coupling member; a convex portion that protrudes from a part of the fitted portion in a radial direction of the distal end barrel, and engages with the recess portion; and an image pickup module that is incorporated in the distal end barrel in a vicinity of the convex portion, and is disposed in a region overlapping the convex portion in the radial direction of the distal end barrel.

### Brief Description of Drawings

Fig. 1 is according to a first embodiment and is a front view of an endoscope.
Fig. 2 is according to the first embodiment and is a front view of a mother-baby endoscope system.
Fig. 3 is according to the first embodiment and is a perspective view showing a distal end portion of the endoscope.
Fig. 4 is according to the first embodiment and is an exploded perspective view showing a distal end barrel and members such as an image pickup module from a rear surface side.
Fig. 5 is according to the first embodiment and is a rear view of the distal end barrel.
Fig. 6 is according to the first embodiment and is a perspective view showing the distal end barrel in which the members such as the image pickup module are assembled, from the rear surface side.
Fig. 7 is according to the first embodiment and is a rear view of the distal end barrel in which the members such as the image pickup module and a coupling ring are assembled.
Fig. 8 is according to the first embodiment and is an exploded perspective view showing the distal end barrel and the coupling ring from the rear surface side.
Fig. 9 is according to the first embodiment and is a perspective view showing the distal end barrel in which the members such as the image pickup module and the coupling ring are assembled, from the rear surface side.
Fig. 10 is according to the first embodiment and is a sectional view of main parts of the distal end portion shown along a longitudinal direction of an insertion portion.
Fig. 11 is according to a first modification example in the first embodiment and is a sectional view of main parts of the distal end portion shown along the longitudinal direction of the insertion portion.
Fig. 12 is according to a second modification example in the first embodiment and is an exploded perspective view showing the distal end barrel and the coupling ring from the rear surface side.
Fig. 13 is according to the second modification example in the first embodiment and is a perspective view showing the distal end barrel in which the members such as the image pickup module and the coupling ring are assembled, from the rear surface side.
Fig. 14 is according to a third modification example in the first embodiment and is a plan view showing the distal end portion in a state where an outer sheath is removed.
Fig. 15 is according to a second embodiment and is an exploded perspective view showing a distal end barrel and a coupling ring from the rear surface side.
Fig. 16 is according to the second embodiment and is a rear view of the distal end barrel in which the members such as the image pickup module and the coupling ring are assembled.
Fig. 17 is according to a first modification example in the second embodiment and is an exploded perspective view showing the distal end barrel and the coupling ring from the rear surface side.

### Best Mode for Carrying Out the Invention

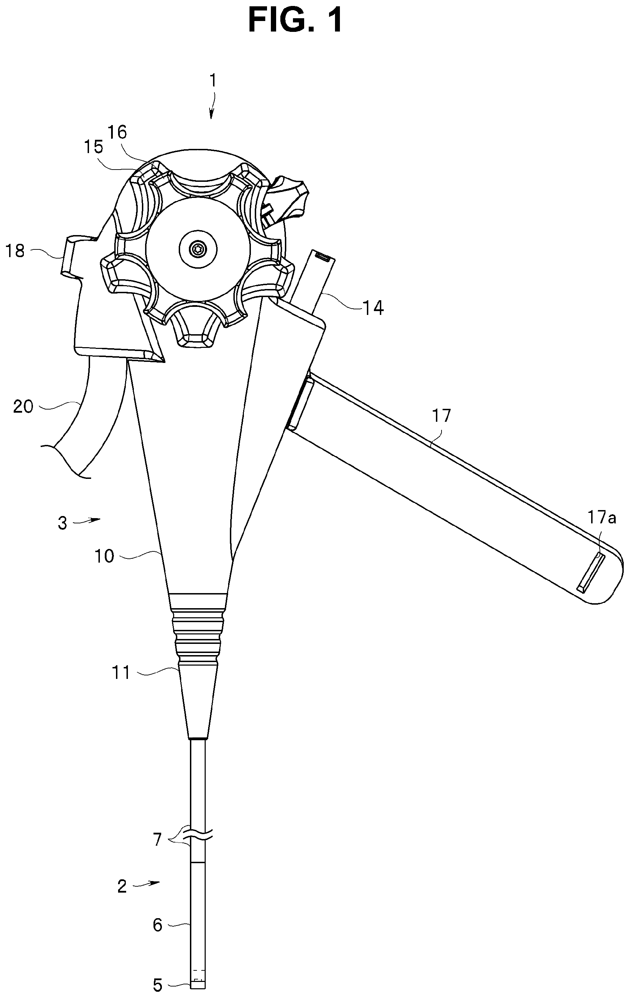

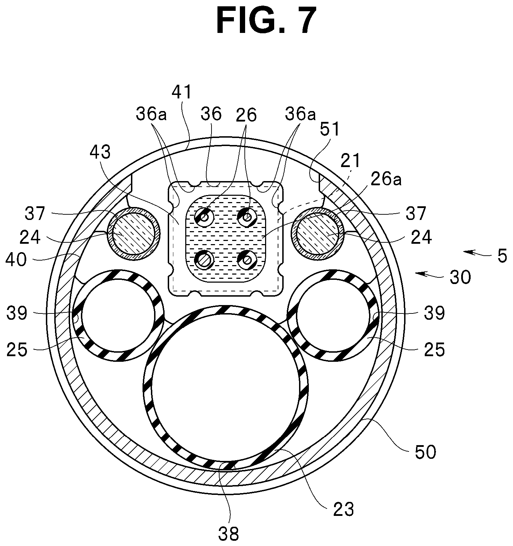

Hereinafter, a first embodiment of the present disclosure will be described with reference to Fig. 1 to Fig. 10.

An endoscope 1 shown in Fig. 1 is a biliopancreatic endoscope for performing observation, treatment, and the like of the bile ducts in the biliary tract or the pancreas. Note that in the present embodiment, the biliopancreatic endoscope is described as an example, but the technology described below can be applied to various other endoscopes for the digestive system, surgery, and the like.

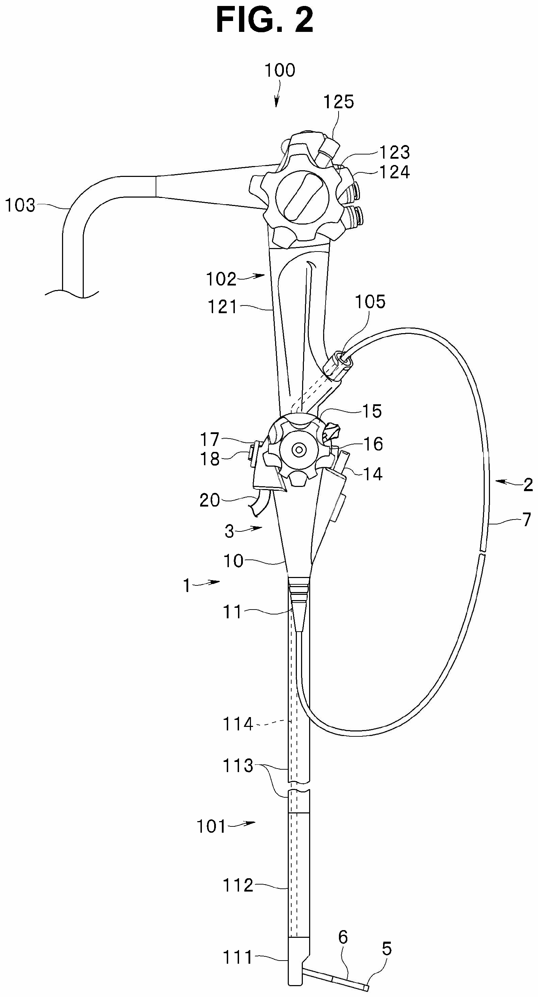

Here, the endoscope 1 of the present embodiment constitutes a mother-baby endoscope with a duodenum endoscope 100 as shown in Fig. 2, for example. In other words, the endoscope 1 is used as a baby endoscope of a mother-baby endoscope system in which the duodenum endoscope 100 is a mother endoscope.

Therefore, prior to describing the endoscope 1 of the present embodiment, a schematic configuration of the duodenum endoscope 100 (hereinafter, referred to as "mother endoscope 100") will be described.

The mother endoscope 100 is, for example, a side-view endoscope. The mother endoscope 100 includes an insertion portion 101, and an operation portion 102 connected to a proximal end of the insertion portion 101.

The insertion portion 101 includes a distal end portion 111 located at a distal end of the insertion portion 101, a bending portion 112 configured to be bendable, and a flexible tube portion 113 having flexibility. The distal end portion 111, the bending portion 112, and the flexible tube portion 113 are connected in order from a distal end side of the insertion portion 2101. Although not shown in the drawings, the distal end portion 111 is provided with an image pickup module, an illumination optical system, a treatment instrument opening, a treatment instrument raising base, and the like.

The mother endoscope 100 further includes a treatment instrument channel 114 provided inside the insertion portion 101 and the operation portion 102. The treatment instrument channel 114 is communicated with a ferrule 105 provided in the operation portion 102, and a treatment instrument opening of the distal end portion 111. The treatment instrument channel 114 is adapted to allow insertion of the insertion portion of the endoscope 1, described later, in addition to treatment instruments such as forceps.

The operation portion 102 includes an operation portion main body 121. The flexible tube portion 113 of the insertion portion 101 is connected to the operation portion main body 121.

The operation portion 102 further includes a plurality of operation members provided in the operation portion main body 121. The plurality of operation members are for operating the mother endoscope 100. The plurality of operation members include two bending operation knobs 123 and 124 for operating bending of the bending portion 112, and a treatment instrument raising lever 125 for operating the treatment instrument raising base provided at the distal end portion 111.

The mother endoscope 100 further includes a universal cord 103 extending from the operation portion 102. The universal cord 103 is connected to an endoscope unit (not shown) for controlling the mother endoscope 100.

Next, a configuration of the endoscope 1 of the present embodiment will be described. The endoscope 1 includes an insertion portion 2, and an operation portion 3 connected to a proximal end of the insertion portion 2.

Here, an outer diameter of the insertion portion 2 is set smaller than an inner diameter of the treatment instrument channel 114 of the mother endoscope 100. Therefore, the insertion portion 2 inserted into the ferrule 105 of the mother endoscope 100 can protrude from the treatment instrument opening of the mother endoscope 100 via the treatment instrument channel 114 of the mother endoscope 100. In addition, the insertion portion 2 can be raised by the treatment instrument raising base provided at the distal end portion 111, when protruding from the treatment instrument opening of the mother endoscope 100 (see Fig. 2).

The insertion portion 2 includes a distal end portion 5 located at a distal end of the insertion portion 2, a bending portion 6 configured to be bendable, and a flexible tube portion 7 having flexibility. The distal end portion 5, the bending portion 6, and the flexible tube portion 7 are connected in order from a distal end side of the insertion portion 2.

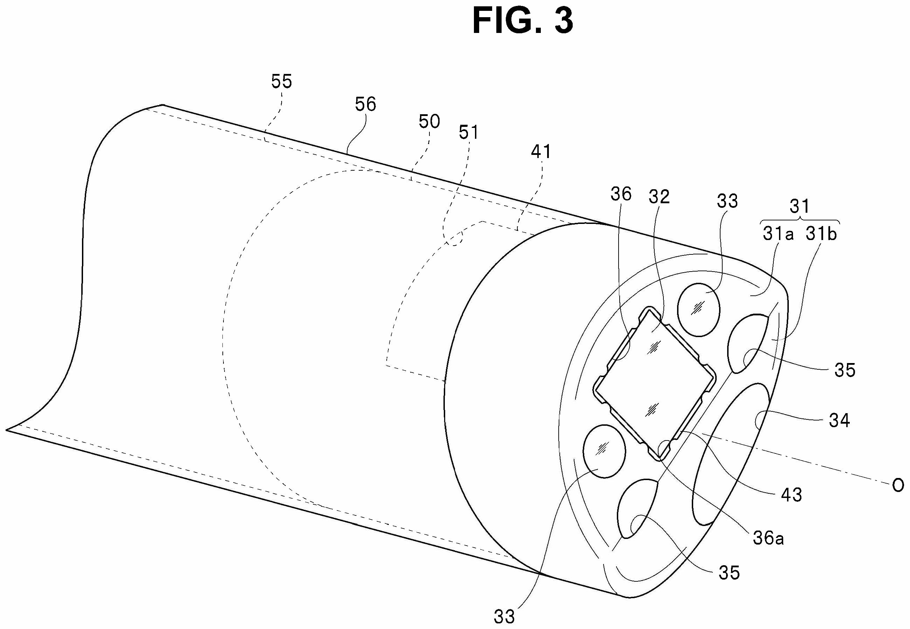

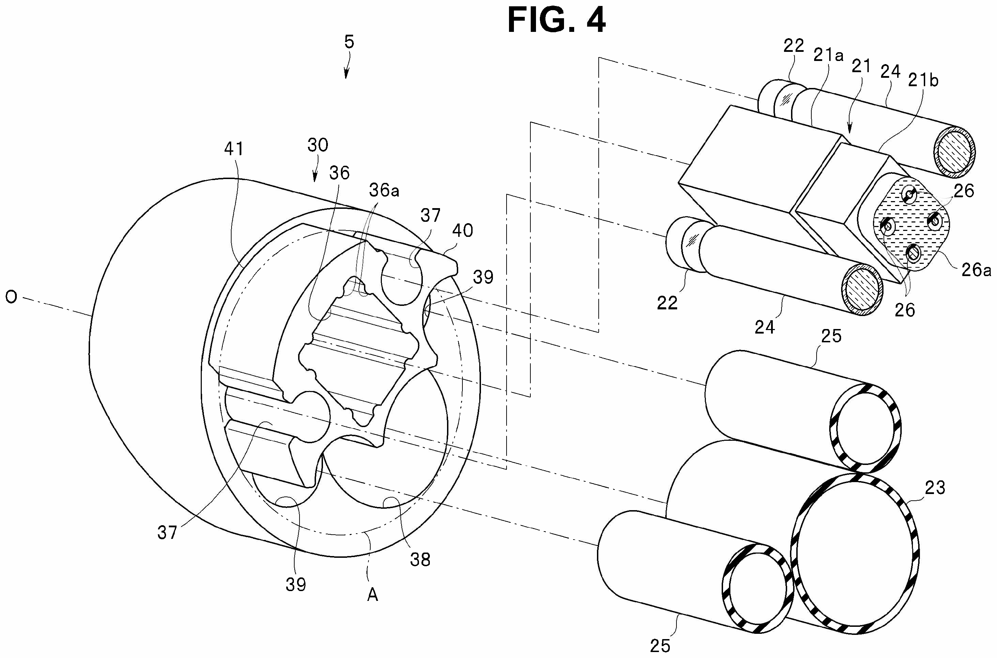

The distal end portion 5 incorporates, for example, an image pickup module 21 and two illumination lenses 22 as shown in Fig. 4. The image pickup module 21 forms an observation window 32 (see Fig. 3) on a distal end surface 31 of the distal end portion 5. In addition, the two illumination lenses 22 form two illumination windows 33 (see Fig. 3) on the distal end surface 31.

In addition, a treatment instrument channel 23, two light guides 24, and two water feeding channels 25 are connected to the distal end portion 5 as shown in Fig. 4, for example. The treatment instrument channel 23 is communicated with a treatment instrument opening 34 (see Fig. 3) provided in the distal end surface 31. The two light guides 24 are optically connected to the two illumination lenses 22, respectively. Furthermore, the two water feeding channels 25 are communicated with two water feeding ports 35 (see Fig. 3) provided in the distal end surface, respectively.

The bending portion 6 includes, for example, a bending tube 55 made of a superelastic alloy (see Fig. 3). The bending tube 55 includes, for example, a plurality of slits (not shown) formed by laser processing. This allows the bending portion 6 to be actively bent in four directions, that is, up, down, left, and right directions. Here, the up, down, left, and right directions of the distal end portion 5 and the bending portion 6 are defined corresponding to up, down, left, and right directions of an endoscopic image picked up by the image pickup module 21.

Note that it is also possible to configure the bending portion 6 using a bending tube in which a plurality of bending pieces are coupled with coupling pins. Alternatively, the bending portion 6 may be configured using a multi-lumen tube.

The flexible tube portion 7 is, for example, configured by a tubular member that is made flexible so as to be passively flexible. Note that the flexible tube portion 7 may be configured using a multi-lumen tube.

The operation portion 3 includes an operation portion main body 10 and a bend preventing portion 11. The flexible tube portion 7 of the insertion portion 2 is connected to the operation portion main body 10. The bend preventing portion 11 covers a connection part of the flexible tube portion 7 to the operation portion main body 10 and the vicinity of the connection part.

The operation portion 3 includes a ferrule 14 provided in the operation portion main body 10. The ferrule 14 is connected to the treatment instrument channel 23 inside the operation portion main body 10.

The operation portion 3 includes a plurality of operation members provided in the operation portion main body 10. The plurality of operation members are for operating the endoscope 1. The plurality of operation members include two bending operation knobs 15 and 16 for operating the bending of the bending portion 6 of the insertion portion 2.

In addition, the operation portion 3 includes a fixing member 17 provided to the operation portion main body 10. The fixing member 17 has a band-like shape that can be wrapped around the operation portion 102 of the mother endoscope 100. One end portion of the fixing member 17 is connected to the operation portion main body 10. The fixing member 17 includes a locking hole 17a in the vicinity of another end portion of the fixing member 17. The locking hole 17a can be locked to a protrusion 18 provided on the operation portion main body 10. Then, in a state where the fixing member 17 is wrapped around the operation portion 102 of the mother endoscope 100, the locking hole 17a is locked to the protrusion 18. This allows the fixing member 17 to fix the endoscope 1 to the mother endoscope 100.

A universal cord 20 is connected to the operation portion 3. The universal cord 20 includes various signal lines 26 extended from the image pickup module 21, the light guides 24, the water feeding channels 25, and the like. The universal cord 20 can be connected to an endoscope unit (not shown) for controlling the endoscope 1.

Next, a configuration of the distal end portion 5 will be described in detail.

The distal end portion 5 includes a distal end barrel 30 formed by a rigid resin, for example. The distal end barrel 30 is a rigid barrel member having a cylindrical shape as a basic shape.

The distal end surface 31 of the distal end barrel 30 includes a first surface 31a perpendicular to a longitudinal axis, and a second surface 31b inclined with respect to the first surface 31a. Therefore, the distal end barrel 30 has a substantially shell shape in particular. Note that the distal end surface 31 can be configured by only the first surface perpendicular to the longitudinal axis.

The distal end barrel 30 includes a plurality of through holes that penetrate the distal end barrel 30 in a longitudinal axis O direction of the insertion portion 2. For example, the distal end barrel 30 includes a first through hole 36, two second through holes 37, a third through hole 38, and two fourth through holes 39.

The first through hole 36 is a through hole for incorporating the image pickup module 21 in the distal end barrel 30. In the present embodiment, the first through hole 36 is configured, for example, as a rectangular hole. A plurality of ribs 36a extending in the longitudinal axis O direction of the insertion portion 2 are provided on an inner wall of the first through hole 36.

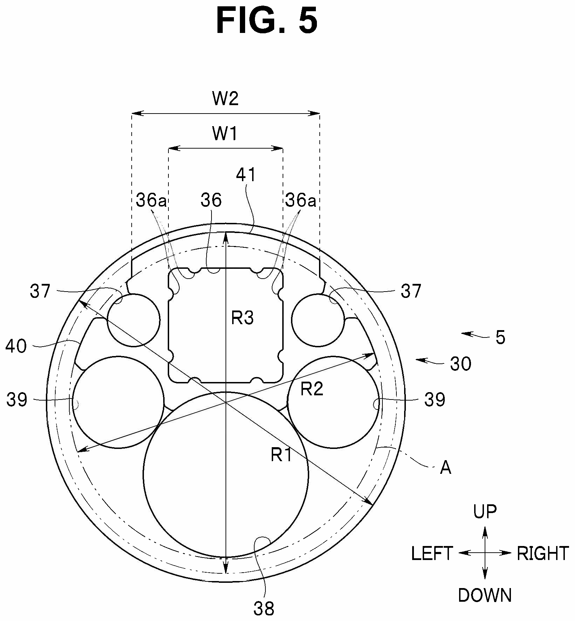

Note that in the following description, up, down, left, and right directions of the distal end barrel 30 are defined corresponding to up, down, left, and right directions of an endoscopic image picked up by the image pickup module 21 (see Fig. 5, for example).

The two second through holes 37 are through holes for incorporating the illumination lenses 22 and distal end parts of the light guides 24 respectively in the distal end barrel 30. In the present embodiment, the second through holes 37 are each configured, for example, as a round hole. The two through holes 37 are disposed, for example, on both left and right sides of the first through hole 36.

The third through hole 38 is a through hole for incorporating a distal end part of the treatment instrument channel 23 in the distal end barrel 30. In the present embodiment, the third through hole 38 is configured, for example, as a round hole. In addition, a distal end of the third through hole 38 is formed as the treatment instrument opening 34. The third through hole 38 is disposed, for example, on a lower side of the first through hole 36. More specifically, the first through hole 36 and the third through hole 38 are arranged vertically in a diameter direction of the distal end barrel 30.

The two fourth through holes 39 are through holes for incorporating distal end parts of the water feeding channels 25 in the distal end barrel 30, respectively. In the present embodiment, the fourth through holes 39 are each configured, for example, as a round hole. In addition, distal ends of the fourth through holes 39 are each formed as the water feeding port 35. The two fourth through holes 39 are disposed, for example, on both left and right sides of the first through hole 36 and the third through hole 38, respectively.

Such a distal end barrel 30 is coupled with the distal end side of the insertion portion 2 via a coupling ring 50 as a coupling member. The coupling ring 50 is adhesively fixed to the distal end barrel 30 in a fitted state on a proximal end side of the distal end barrel 30.

For this reason, the distal end barrel 30 includes a fitted portion 40 for fitting the coupling ring 50 on the proximal end side. An outer diameter dimension R2 of the fitted portion 40 is set smaller than an outer diameter dimension R1 of the distal end barrel 30 on the distal end side.

In other words, the fitted portion 40 is provided inside of a circular region A (see two-dot chain lines in Figs. 4, 5, and 8, for example) having the outer diameter dimension R2 smaller than the outer diameter dimension R1 of the distal end barrel 30 on the distal end side. In the present embodiment, more specifically, the fitted portion 40 is provided in a region inside and on an upper side of the circular region A, on the proximal end side of the distal end barrel 30. At least the first through hole 36 is formed in the region where the fitted portion 40 is provided. Here, the outer diameter dimension R2 of the circular region A is substantially equal to an inner diameter dimension of the coupling ring 50.

In addition, the distal end barrel 30 includes a convex portion 41 that protrudes from a part of the fitted portion 40 in an outer diameter direction of the distal end barrel 30. Specifically, the convex portion 41 protrudes from a part of an upper side of the fitted portion 40 toward the outer diameter direction of the distal end barrel 30. Therefore, the convex portion 41 is disposed at a position in line with the first through hole 36 and the third through hole 38 in a radial direction of the distal end barrel 30. In other words, the first through hole 36 is disposed between the third through hole 38 and the convex portion 41 in the radial direction of the distal end barrel 30.

In the present embodiment, a rear view shape of the convex portion 41 is a substantially arc-shape. Here, an outer diameter dimension R3 of the convex portion 41 having the substantially arc-shape is substantially equal to an outer diameter dimension of the coupling ring 50.

In addition, when the distal end barrel 30 is viewed from above in a plan view, a width W2 of the convex portion 41 is set greater than a width W1 of the first through hole 36. Therefore, even when the first through hole 36 is disposed at a position close to an upper side of the distal end barrel 30, the inner wall of the first through hole 36 is formed over the entire circumference without being interrupted also on the proximal end side of the distal end barrel 30 (fitted portion 40).

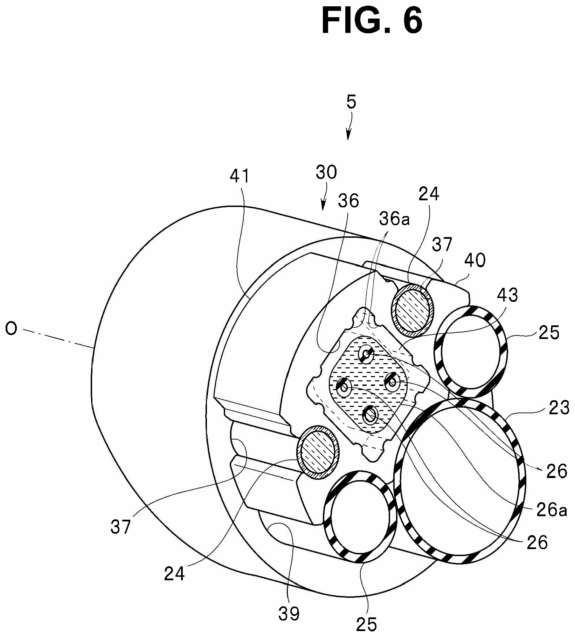

In the distal end barrel 30 formed in this manner, the image pickup module 21 is inserted into the first through hole 36 as shown in Figs. 6, 7, and 10, for example.

Here, main parts of the image pickup module 21 of the present embodiment are configured by a CSP (chip size package) in which the lens unit 21a and the image pickup device 21b are integrally stacked and packaged as shown in Fig. 4, for example. The lens unit 21a is configured by a lens stack fabricated using a wafer-level optics technology, or the like. In other words, the lens unit 21a is manufactured by stacking a plurality of lens wafers in each of which a lens is formed on a base material such as a glass substrate, for example, and then dicing the stacked lens wafers. Therefore, the lens unit 21a has a rectangular shape in a plan view, and does not include a lens barrel.

When the image pickup module 21 is inserted into the first through hole 36, the plurality of ribs 36a provided on the inner wall of the first through hole 36 guide the image pickup module 21. Furthermore, the plurality of ribs 36a form gaps between the first through hole 36 and the image pickup module 21. The gaps between the first through hole 36 and the image pickup module 21 are filled with an adhesive 43. Therefore, the image pickup module 21 is adhesively fixed to the first through hole 36 in a liquid-tight manner.

Here, a plurality of signal lines 26 (four in this case) are connected to a rear surface of the image pickup device 21b. Distal end sides of the signal lines 26 are covered by a protective member 26a formed of an adhesive or the like on the rear surface side of the image pickup device 21b. In the present embodiment, distal end parts of the signal lines 26 covered by the protective member 26a are inserted into the first through hole 36 as a part of the image pickup module 21.

The illumination lenses 22 are inserted into the second through holes 37, respectively. The illumination lenses 22 are fixed to the distal end barrel 30 using an adhesive or the like on a distal end side of the second through holes 37, respectively. By fixing the illumination lenses 22, the distal end sides of the second through holes 37 are blocked in a liquid-tight manner.

Furthermore, the distal end parts of the light guides 24 are inserted into the second through holes 37, respectively. The light guides 24 are fixed to the distal end barrel 30 using an adhesive or the like in contact with the illumination lenses 22, respectively. Therefore, the light guides 24 are connected to the distal end barrel 30 in a state where the light guides 24 are optically connected to the illumination lenses 22, respectively.

The distal end part of the treatment instrument channel 23 is inserted into the third through hole 38. The distal end part of the treatment instrument channel 23 is fixed to the distal end barrel 30 using an adhesive or the like. Therefore, the treatment instrument channel 23 is connected to the distal end barrel 30 in a state where the treatment instrument channel 23 communicates with the treatment instrument opening 34 formed on a distal end side of the third through hole 38.

The distal end parts of the water feeding channels 25 are inserted into the fourth through holes 39, respectively. The distal end parts of the water feeding channels 25 are fixed to the distal end barrel 30 using an adhesive or the like. Therefore, the water feeding channels 25 are connected to the distal end barrel 30 in a state where the water feeding channels 25 communicate with the water feeding ports 35 formed on distal end sides of the fourth through holes 39, respectively.

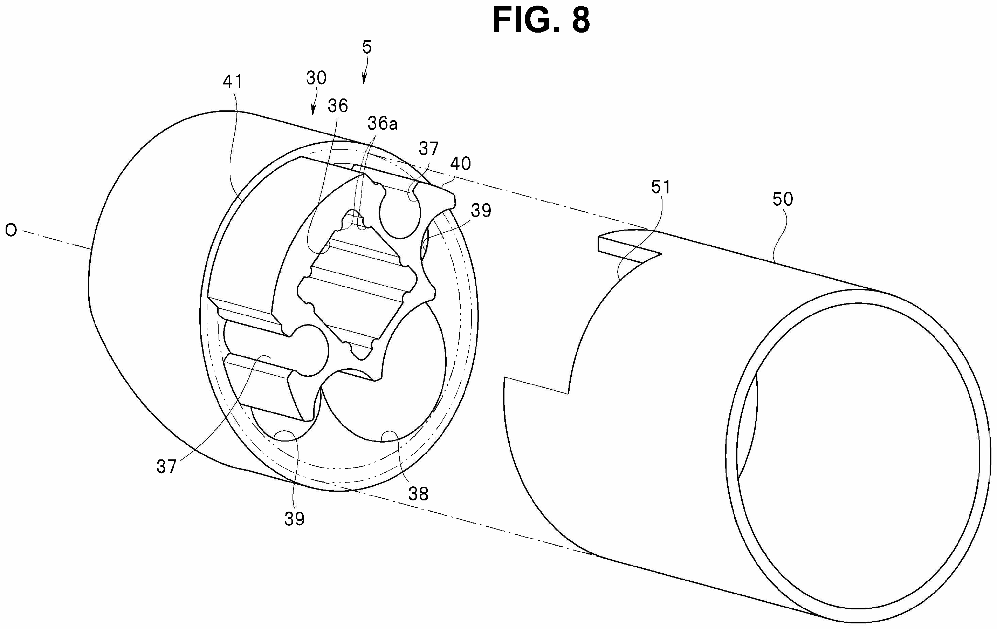

As shown in Fig. 8, the coupling ring 50 includes a recess portion 51 on a distal end side thereof. The recess portion 51 is configured by a cutout provided in a part in a circumferential direction on the distal end side of the coupling ring 50. The recess portion 51 is provided to avoid interference with the convex portion 41 when the coupling ring 50 is fitted into the fitted portion 40 of the distal end barrel 30.

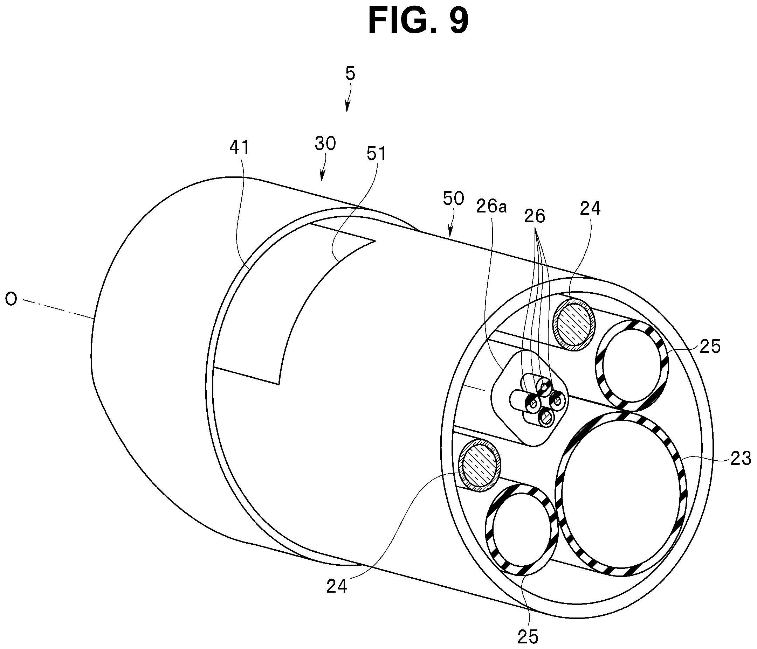

For example, as shown in Fig. 9, the distal end side of the coupling ring 50 is fitted into the fitted portion 40 of the distal end barrel 30 in a state where the recess portion 51 is engaged with the convex portion 41. Then, the distal end side of the coupling ring 50 is fixed to the fitted portion 40 using an adhesive or the like.

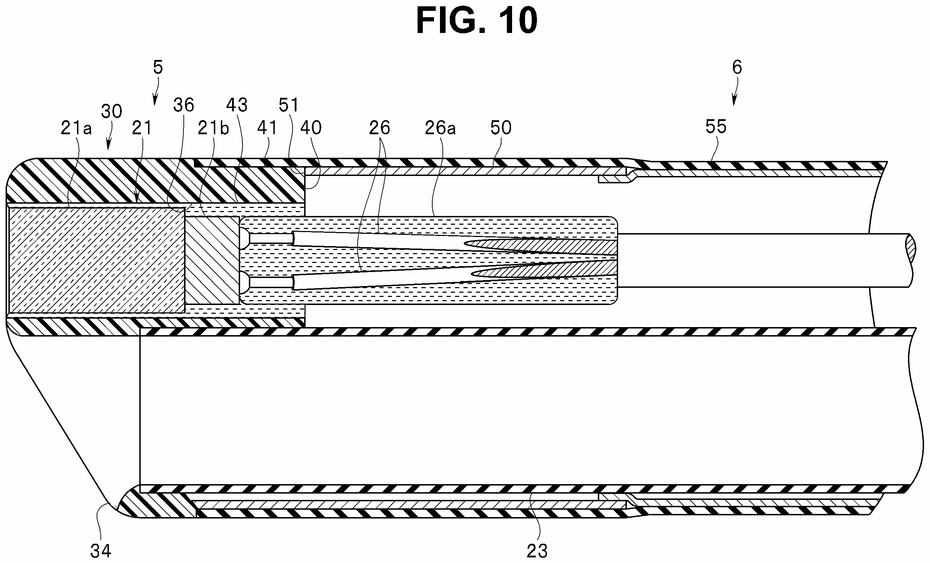

In addition, for example, as shown in Fig. 10, a proximal end side of the coupling ring 50 is fitted into a distal end side of the bending tube 55. Then, the proximal end side of the coupling ring 50 is fixed to the bending tube 55 using an adhesive or the like.

In this manner, the distal end barrel 30 (distal end portion 5) is coupled with the bending portion 6 of the insertion portion 2 via the coupling ring 50.

Here, for example, as shown in Figs. 3 and 10, outer circumferences of the coupling ring 50 and the bending tube 55 are covered by an outer sheath 56 formed of a resin or the like.

Note that, for example, when the bending portion 6 is configured using the bending tube 55 made of a superelastic alloy, the bending tube 55 may also be used as the coupling ring 50. In this case, the recess portion 51 is provided on the distal end side of the bending tube 55 to avoid the interference with the convex portion 41. Alternatively, for example, when the bending portion 6 is configured using a bending tube in which a plurality of bending pieces are coupled with coupling pins, a bending piece located at a distal-most end may also be used as the coupling ring 50. In this case, the recess portion 51 is provided on a distal end side of the bending piece at the distal-most end to avoid the interference with the convex portion 41.

According to such an embodiment, the endoscope 1 includes the distal end barrel 30, the fitted portion 40 that is provided on the proximal end side of the distal end barrel 30 and has the outer diameter dimension smaller than that of the distal end barrel 30 on the distal end side, the coupling ring 50 fitted into the fitted portion 40, the recess portion 51 provided in the coupling ring 50, the convex portion 41 that protrudes from the part of the fitted portion 40 in the radial direction of the distal end barrel 30 and engages with the recess portion 51, and the image pickup module 21 that is incorporated in the distal end barrel 30 in the vicinity of the convex portion 41 and disposed in the region overlapping the convex portion 41 in the radial direction of the distal end barrel 30.

This allows the image pickup module 21 to be adhesively fixed to the distal end barrel 30 accurately without increasing the overall outer diameter dimension of the distal end barrel 30.

In other words, the distal end barrel 30 includes the convex portion 41 that protrudes from the part of the fitted portion 40 into which the coupling ring 50 is fitted, in the radial direction of the distal end barrel 30. By providing such a convex portion 41, it is possible to expand a region for disposing each member such as the image pickup module 21 and the like in the distal end barrel 30, without increasing the outer diameter dimension of the fitted portion 40 (and, consequently, without increasing the overall outer diameter dimension of the distal end barrel 30). The first through hole 36 for incorporating the image pickup module 21 in the distal end barrel 30 is then disposed in the region that is the vicinity of the convex portion 41 and overlaps the convex portion 41 in the radial direction of the distal end barrel 30. Thus, even when the first through hole 36 is disposed at a position close to an end (upper side) of the distal end barrel 30, the inner wall of the first through hole 36 can be formed over the entire circumference without being interrupted also on the proximal end side of the distal end barrel 30 (fitted portion 40). This makes it possible to reduce a requirement for a skill of the worker when adhesively fixing the image pickup module 21 to the first through hole 36. It also makes it possible to reduce a requirement for a skill of the worker when positioning the optical axis direction or the like of the image pickup module 21 along a wall surface of the first through hole 36.

In this case, the coupling ring 50 includes the recess portion 51 that engages with the convex portion 41. The recess portion 51 allows the coupling ring 50 to avoid the interference with the convex portion 41 when the coupling ring 50 is fitted into the fitted portion 40.

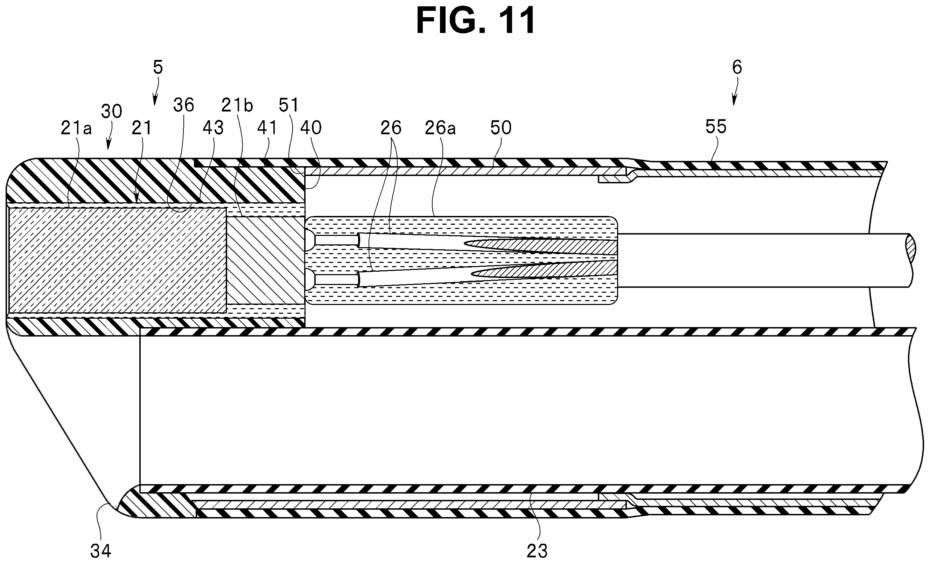

Here, for example, as shown in Fig. 11, the image pickup module 21 inserted into the first through hole 36 may be configured so as not to include the distal end sides of each signal line 26 and the protective member 26a.

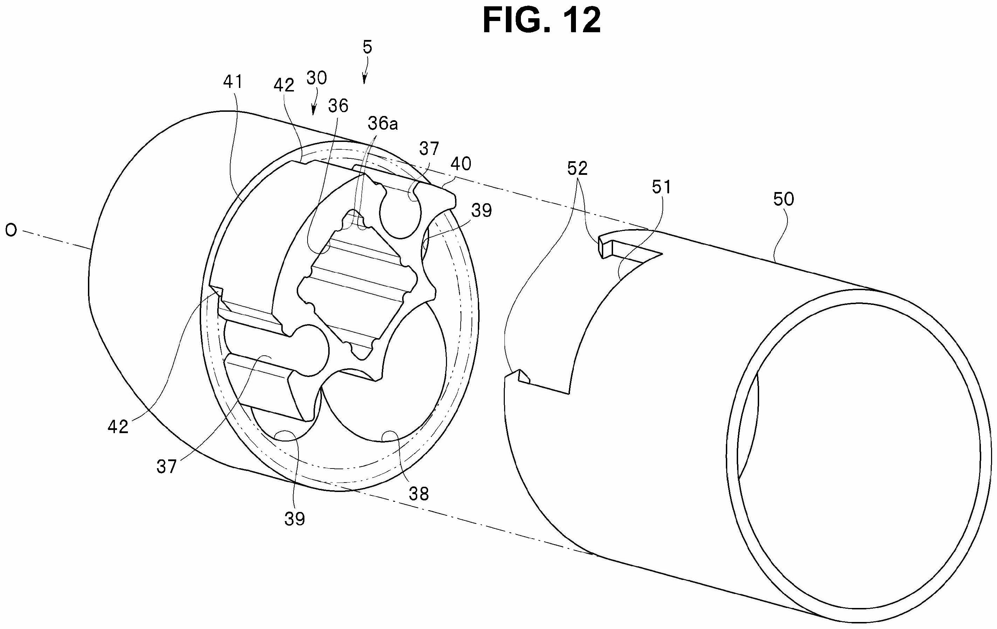

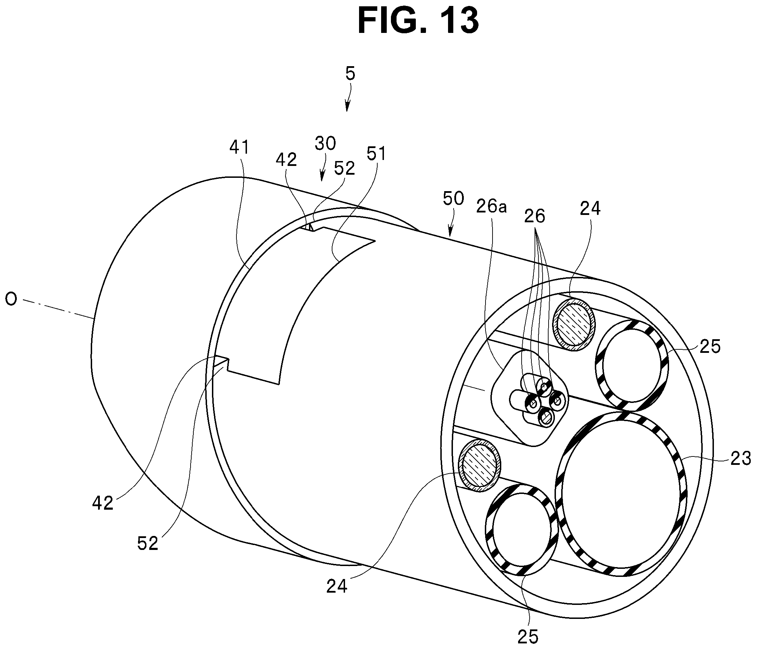

In addition, for example, as shown in Figs. 12 and 13, when the coupling ring 50 is fitted into and adhered to the fitted portion 40 of the distal end barrel 30, an engagement mechanism may be provided for temporarily fixing the coupling ring 50 to the distal end barrel 30.

The engagement mechanism of the present modification example includes a pair of claw portions 52 provided in the recess portion 51 of the coupling ring 50, and a pair of groove portions 42 provided in the convex portion 41.

Each of the claw portions 52 is provided in a state protruding in the circumferential direction of the coupling ring 50. In addition, each of the groove portions 42 is provided in a state recessed in a circumferential direction of the convex portion 41.

The claw portions 52 engage with the groove portions 42 respectively when the coupling ring 50 is fitted into the fitted portion 40. In this manner, the coupling ring 50 is temporarily fixed to the distal end barrel 30. Then, in a state where the coupling ring 50 is temporarily fixed to the distal end barrel 30 in this manner, the coupling ring 50 is fixed to the distal end barrel 30 using an adhesive or the like. Therefore, a fitting state of the coupling ring 50 into the distal end barrel 30 can be accurately maintained until the adhesive or the like hardens.

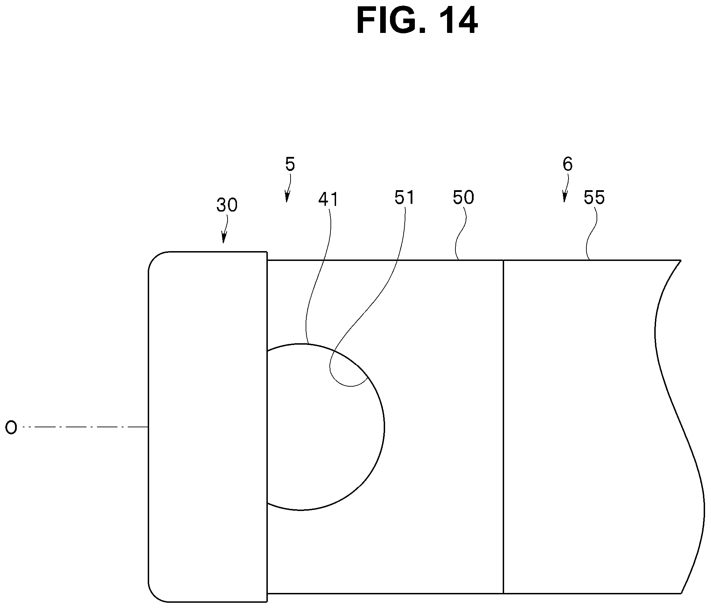

In addition, for example, as shown in Fig. 14, plan-view shapes of the convex portion 41 and the recess portion 51 when viewed from above of the distal end portion 5 may be made partially circular. In this case, by making the convex portion 41 and the recess portion 51 partially circular with an arc length longer than an arc length of a semicircle, the coupling ring 50 can be temporarily fixed to the distal end barrel 30 without providing the claw portions 52 and the groove portions 42 described above.

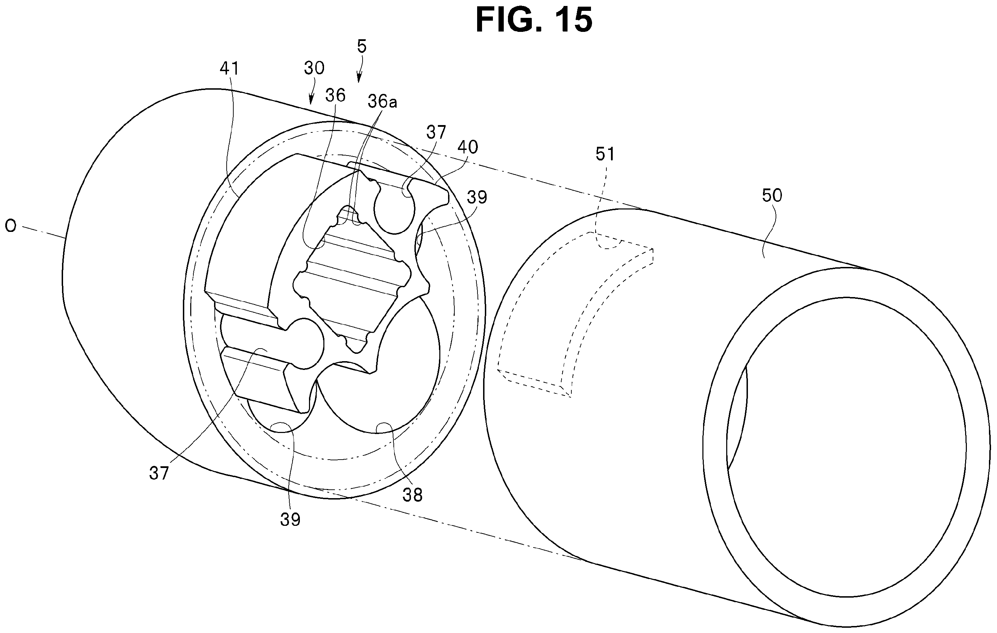

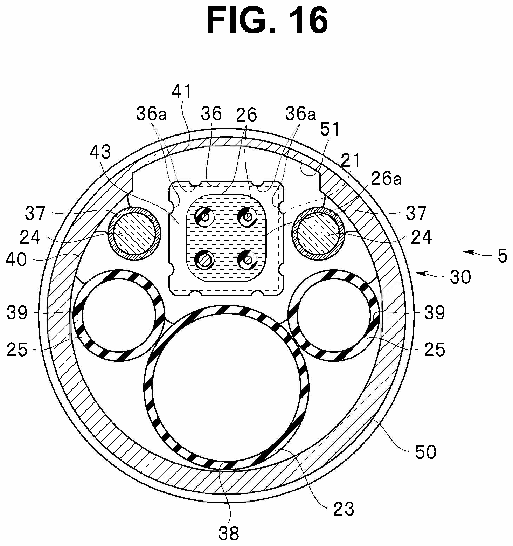

Next, a second embodiment of the present disclosure will be described with reference to Figs. 15 and 16. Here, the present embodiment differs from the first embodiment mainly in a form of the recess portion 51 provided in the coupling ring 50. Other configurations that are the same as those in the first embodiment are added with the same reference signs and descriptions thereof are omitted.

Note that in particular, the present embodiment aims at improving a coupling strength of the distal end barrel 30, and can be applied advantageously to the endoscope 1 employing a thick coupling ring 50, or the like.

As shown in Figs. 15 and 16, a recess portion 51 of the present embodiment is configured by an arc-shaped recess portion formed by recessing an inner circumferential surface of the coupling ring 50 in the radial direction. When fitting the coupling ring 50 into the fitted portion 40 of the distal end barrel 30, the convex portion 41 protruding from the fitted portion 40 is engaged with the recess portion 51.

According to such an embodiment, the effects as in the first embodiment described above can be achieved.

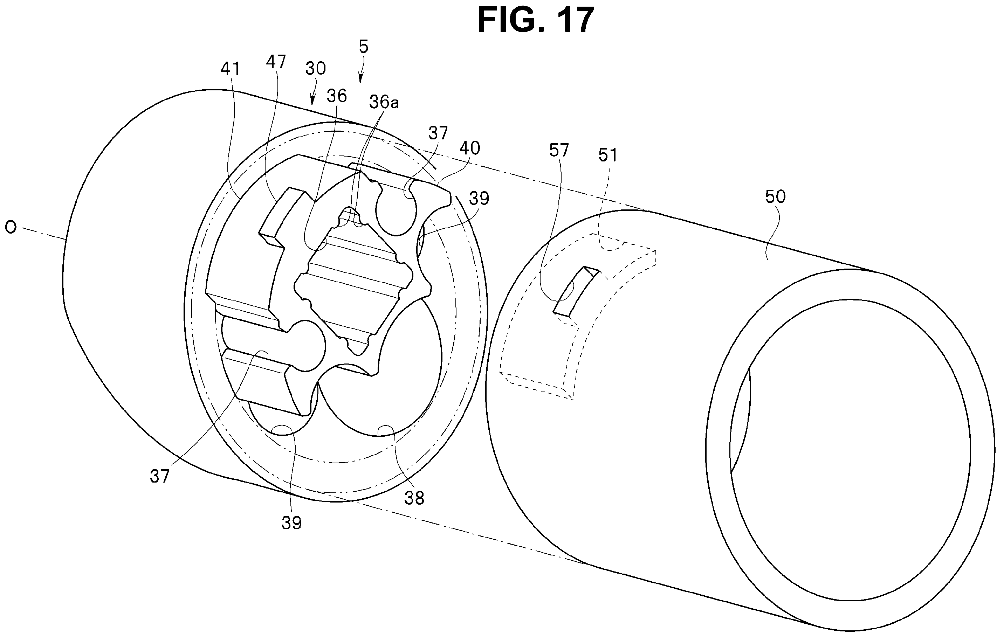

Here, for example, as shown in Fig. 17, when the coupling ring 50 is fitted into and adhered to the fitted portion 40 of the distal end barrel 30, an engagement mechanism for temporarily fixing the coupling ring 50 to the distal end barrel 30 can be provided.

The engagement mechanism of the present modification example includes a claw portion 47 provided in the convex portion 41, and a groove portion 57 provided in the recess portion 51 of the coupling ring 50.

The claw portion 47 is provided in a state protruding in a radial direction of the convex portion 41. In addition, the groove portion 57 is provided in a state further recessed from the recess portion 51 of the coupling ring 50 in the radial direction. Note that in the modification example, the groove portion 57 penetrates the coupling ring 50 in the radial direction.

The claw portion 47 engages with the groove portion 57 when the coupling ring 50 is fitted into the fitted portion 40. Therefore, the coupling ring 50 is temporarily fixed to the distal end barrel 30. Then, in a state where the coupling ring 50 is temporarily fixed to the distal end barrel 30 in this manner, the coupling ring 50 is fixed to the distal end barrel 30 using an adhesive or the like. Therefore, a fitting state of the coupling ring 50 into the distal end barrel 30 can be accurately maintained until the adhesive or the like hardens.

Note that the present technology is not limited to the embodiments described above, and various modifications and applications are possible, which are also within the technical scope of the present technology. In addition, it goes without saying that the configurations of the above embodiments and modification examples may be combined as appropriate.

This application is based on and claims priority to U.S. Provisional Application No. 63/538160, filed in the United States on September 13, 2023, the entire contents of which are incorporated into the specification, claims, and drawing of this application by reference.

## Claims

1. An endoscope comprising:
a distal end barrel constituting a distal end portion of an insertion portion;
a fitted portion that is provided on a proximal end side of the distal end barrel and has an outer diameter dimension smaller than an outer diameter dimension on a distal end side of the distal end barrel;
a coupling member fitted into the fitted portion;
a recess portion provided in the coupling member;
a convex portion that protrudes from a part of the fitted portion in a radial direction of the distal end barrel, and engages with the recess portion; and
an image pickup module that is incorporated in the distal end barrel in a vicinity of the convex portion, and is disposed in a region overlapping the convex portion in the radial direction of the distal end barrel.

2. The endoscope according to claim 1, wherein the coupling member is an annular member.

3. The endoscope according to claim 2, wherein the recess portion is a cutout provided at a part in a circumferential direction of the coupling member.

4. The endoscope according to claim 2, wherein the recess portion is a recess formed in a thickness direction of the coupling member.

5. The endoscope according to claim 1, wherein the image pickup module is disposed between a channel provided in the distal end barrel and the convex portion, in the radial direction of the distal end barrel.

6. The endoscope according to claim 1, wherein a width of the convex portion is greater than a width of the image pickup module.

7. The endoscope according to claim 1, wherein the coupling member couples the distal end barrel with a distal end side of the insertion portion.

8. The endoscope according to claim 1, wherein:
the coupling member includes a claw portion in the recess portion; and
the distal end barrel includes, in the convex portion, a groove portion for engaging with the claw portion.

9. The endoscope according to claim 8, wherein:
the claw portion is provided in a circumferential direction of the coupling member; and
the groove portion is provided in a circumferential direction of the distal end barrel.

10. The endoscope according to claim 1, wherein:
the distal end barrel includes a claw portion in the convex portion; and
the coupling member includes, in the recess portion, a groove portion for engaging with the claw portion.

11. The endoscope according to claim 10, wherein:
the claw portion is provided in the radial direction of the distal end barrel; and
the groove portion is provided in a radial direction of the recess portion.

12. The endoscope according to claim 8, wherein the recess portion has a plan-view shape in which a part of the coupling member is cut out into a partial circular shape so as to leave an arc length longer than an arc length of a semicircle arc.

13. The endoscope according to claim 1, wherein the image pickup module includes a lens unit and an image pickup device.

14. The endoscope according to claim 1, wherein the image pickup module includes a lens unit, an image pickup device, and a distal end part of a signal line connected to the image pickup device.
